# EUROPEAN PATENT SPECIFICATION

(11) **EP 3 764 983 B1**
(45) Date of publication and mention of the grant of the patent: **01.09.2021**
(21) Application number: 18830015.6
(22) Date of filing: 13.04.2018
(51) Int. Cl.: A61K 9/00, A61K 47/32, A61K 9/48, A61K 9/50

(54) **A SUSTAINED RELEASE FORMULATION COMPRISING ACEMETACIN WITH BIMODAL IN VITRO RELEASE**
FORMULIERUNG MIT VERZÖGERTER FREISETZUNG MIT ACEMETACIN MIT BIMODALER IN-VITRO-FREISETZUNG
FORMULATION À LIBÉRATION PROLONGÉE COMPRENANT DE L'ACÉMÉTACINE À LIBÉRATION IN VITRO BIMODALE

(43) Date of publication of application: 20.01.2021
(73) Proprietor: Santa Farma Ilaç Sanayi A.S., 34382 Istanbul (TR)
(72) Inventor: YILDIRIM, Ersin, 41455 Dilovas /Kocaeli (TR); KANIK, Bayram, 41455 Dilovas /Kocaeli (TR); AKTAS, Tansel, 41455 Dilovas /Kocaeli (TR); ÖZTÜRK, Fatma, 41455 Dilovas /Kocaeli (TR)
(74) Representative: Bulut, Pinar
(86) International application number: PCT/TR2018/050165
(87) International publication number: WO 2019/199246

(56) References cited:
- WO-A2-2008/062475
- MX-A- 2016 017 001
- US-A1- 2008 213 339

## Description

### Field of the Invention

The present invention relates to a novel pharmaceutical formulation which provides sustained release in multiple-unit dosage form by bimodal in vitro release comprising acemetacin or a pharmaceutically acceptable salt thereof Also disclosed is such a formulation for use in the treatment of pain and inflammation associated with problems in the musculoskeletal system.

### Background of the Invention

Acemetacin is a non-steroid anti-inflammatory drug in indomethacin glycolic acid ester structure. Acemetacin is used in the treatment of pain and inflammation after surgery with discomforts related to the musculoskeletal system and joints. It has a broad treatment area and is also used in osteoarthritis and rheumatoid arthritis indications.

The pharmacological activity of acemetacin is dependent both on acemetacin and indomethacin, which is the main metabolite. The chemical name of acemetacin is O-((1-p-chlorobenzoyl-5-methoxy-2-methylindole-3-yl) acetyl) glycolic acid and its chemical structure is as shown in Formula 1.

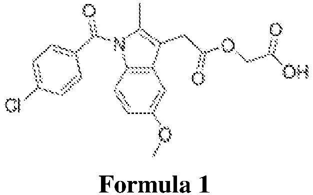

Daily dose for oral administration of acemetacin is between 120-180 mg. This dose is given in divided doses. In case of administration at 60 mg dose, it reaches maximum plasma concentrations in 2 - 2.5 hours. According to British Pharmacopoeia, acemetacin is practically insoluble in aqueous solutions at acidic pH, soluble in acetone and slightly soluble in anhydrous ethanol. Moreover, it has also been reported that solubility of acemetacin decreases rapidly in the acidic medium (1.95 g L⁻¹ solubility at pH 7.4, 23 mg L⁻¹ solubility at pH 5.0).

Acemetacin is a BCS II prodrug which presents low solubility, high permeability characteristics. Formulation development studies of prodrugs that belongs to BCS II should be performed sensitively. One disadvantage of these studies is decreasing solubility characteristics of the prodrug. Therefore, dissolution profile of the drug is a challenging process.

Acemetacin is in the market with authorisation holder of Meda Pharma and Rantudil Forte^{®} trademark which provides immediate release in capsule form (60 mg) and Rantudil Retard^{®} trademark which provides modified release in capsule form (90 mg).

Rantudil Retard^{®} capsule comprising 90 mg acemetacin as active substance. It is indicated in the treatment of symptoms and signs of osteoarthritis, rheumatoid arthritis and ankylosing spondylitis, acute gout arthritis, acute musculoskeletal pain, postoperative pain and dysmenorrhea treatment.

Rantudil Retard^{®} capsule formulation is composed of gelatin, lactose monohydrate, magnesium stearate, sodium dodecyl sulphate, povidone 25, crospovidone, colloidal anhydrous silica, talc, iron oxides in different colors and titanium dioxide.

In the prior art, US4900557A numbered patent application that belongs to Troponwerke discloses a formulation provides sustained release whcih is developped to prolong the duration of acemetacin being in gastrointestinal tract and comprises pellet. In the scope of this, density of the pellets are increased with a lacquer (cellulose phthalate, HPMC phthalate, other cellulose derivatives, metacrylic acid and metacrylate ester polymer or copolymers). In the description of the patent, density is increased with film coating agent as targetted and it is reported that it is achieved with the density range of 1.4 to 2.4 g/cm³.

In the prior art, US20170105966A1 numbered patent application discloses a formulation comprising acemetacin in micronised form by mixing preferably with flavonoid derivative like neohesperidine or a polypeptide like thaumatine and/or indomethacin. In the present invention, masking of bitter taste is intended.

In the prior art, US6599529B1 numbered patent application discloses a multiple-unit dosage form comprising both modified and immediate release and non-steroidal anti-inflammatory drug. In the mentioned form, there are two units in which one of them provides immediate release unit by alkaline or antacid agents, another unit provides retard release by water-insoluble coating which disintegrates in water and independet of pH. In the prior art, MX2016017001 discloses formulations comprising acemetacin with bimodal release.

In particular with the present invention, a suitable dissolution profile is targeted for a formulation containing acemetacin which exhibits low solubility in acidic media (such as stomach pH). The participation of the active substance in the bloodstream (absorption of the active substance) depends on the dissolution profile. Therefore, the rapid onset of action of the drug in the acidic environment ensures that the initial absorption starts immediately and thus achieves a rapid therapeutic effect.

In the present invention, a bimodal in vitro release formulation comprising acemetacin, both required to have a rapid action and a retard release, has been developed. A continuous effect has been achieved with the initiation of the therapeutic effect quickly and accurately. For further process, a formulation has been developed that provides an improved side effect profile and improved patient compliance, taking one or two doses per day for the treatment of post-operative pain and inflammation associated with musculoskeletal and joint complaints.

### Brief description of the Invention

The invention relates to an oral pharmaceutical formulation with bimodal in vitro release comprising acemetacin or a pharmaceutically acceptable salt thereof wherein the composition is composed of a. an immediate-release unit comprising micronised acemetacin or its pharmaceutically acceptable salt thereof, wherein the particle size of micronized acemetacin is d(90) 5 µm or less, d(50) 1 to 3 µm, d(10) less than 1 µm, b. a retard-release unit comprising non-micronised acemetacin or its pharmaceutically acceptable salt thereof and at least two release modifiers, wherein the particle size of non-micronized acemetacin is d(90) 100 µm or less, d(50) 10 to 20 µm, d(10) less than 5 µm, wherein the multiple-unit dosage form presents a dissolution profile at least 20% after 15 minutes and 30%-40% after one hour in conditions of Type I apparatus (basket), 100 rpm at 37°C, 900 ml USP buffer pH 5.0 with the w/w ratio of non-micronised acemetacin to micronised acemetacin of 2:1 to 5:1. Also disclosed is a sustained-release oral pharmaceutical formulation comprising acemetacin or a pharmaceutically acceptable salt thereof, for use in the treatment of pain and inflammation associated with problems in the musculoskeletal system, to provide additional advantages to the relevant technique.

Also disclosed is a sustained-release oral pharmaceutical formulation comprising acemetacin or a pharmaceutically acceptable salt thereof, wherein both a rapid onset of action and a retard release is required.

An object of this invention is to provide sustained release pharmaceutical formulation with a bimodal in vitro release comprising acemetacin or a pharmaceutically acceptable salt thereof, which comprises retard release unit and immediate release unit.

A further object of the present invention is to provide a pharmaceutical formulation which sustains therapeutic efficacy due to retard release of acemetacin by inhibition of its solubility in stomach and allow to release at a specific dissolution rate in the intestine. In this way, it is possible to avoid possible side effects that may occur in stomach.

Another objective of the present invention is to develop a sustained release pharmaceutical formulation having an in vitro dissolution profile in which the the first unit presents immediate release and second unit presents retard release.

A further object of the present invention is to develop a sustained release pharmaceutical composition which provides an in vitro dissolution profile, an effective amount of at least 20% of the active ingredient over the first 15 minutes and between 30% and 40% after 1 hour is released when tested using the USP apparatus I (basket) at 100 rpm for 60 minutes at 37°C by using pH 5.0 USP buffer dissolution medium.

Also disclosed is a manufacturing method for the formulation of acemetacin.

### Detailed description of the invention

The present invention is related to an oral pharmaceutical formulation with bimodal in vitro release comprising acemetacin or a pharmaceutically acceptable salt thereof wherein the composition is composed of a. an immediate-release unit comprising micronised acemetacin or its pharmaceutically acceptable salt thereof, wherein the particle size of micronized acemetacin is d(90) 5 µm or less, d(50) 1 to 3 µm, d(10) less than 1 µm, b. a retard-release unit comprising non-micronised acemetacin or its pharmaceutically acceptable salt thereof and at least two release modifiers, wherein the particle size of non-micronized acemetacin is d(90) 100 µm or less, d(50) 10 to 20 µm, d(10) less than 5 µm, wherein the multiple-unit dosage form presents a dissolution profile at least 20% after 15 minutes and 30%-40% after one hour in conditions of Type I apparatus (basket), 100 rpm at 37°C, 900 ml USP buffer pH 5.0 with the w/w ratio of non-micronised acemetacin to micronised acemetacin of 2:1 to 5:1. Also disclosed is a sustained release bimodal oral formulation comprising acemetacin or a pharmaceutically acceptable salt for use in the treatment of pain and inflammation due to discomforts in the musculoskeletal system and the manufacturing method for this composition.

The present pharmaceutical formulation providing a sustained release has a dosage form comprising multiple units.

The present "dosage form" is a form having a formulation containing the active ingredient or ingredients in an amount sufficient to provide a therapeutic effect as a result of a single administration.

The "multiple unit" is the multiple unit compositions in single dosage form. Multi unit dosage form contains more than one units. Preferably, the particle is used as a pellet, a granule, a bead, a mini tablet, or a mixture of at least two of them. Units can exhibit the same or different release characteristics. The multiple units are placed in a dosage form of gelatin capsule or compressed as a tablet.

In a preferred embodiment of the invention, the pharmaceutical formulation as a multiple-unit dosage form comprises multiple units exhibiting different release characteristics.

The subject invention is a sustained release pharmaceutical formulation comprising acemetacin or its pharmaceutically acceptable salt thereof, which presents bimodal in vitro release is composed of multiple units as immediate release unit and retard release unit.

In the present invention, the sustained release formulation is a pharmaceutical formulation that maintains stable drug levels in the bloodstream of a patient by releasing the drug over a period of time. The formulation is formulated to release the active ingredient slowly and preferably within about 6 hours. It is possible to obtain a better side effect profile with retard release form in comparison that formed by the release of the drug totally in stomach.

In the present invention, "retard release unit" is the pharmaceutical formulation disintegrates with a modified dissolution rate and dissolves to release the drug after a dedicated time.

In the present invention, "retard release" term is used as similar to controlled release, extended or prolonged release, slow release, long acting release terms.

In the present invention, achieving the desired release rates in the said pharmaceutical formulation is related to the content and the proportions of the formulation. In summary, important factors in the development of a retard release oral pharmaceutical formulation comprising acemetacin or a pharmaceutically acceptable salt thereof are, the use of micronized and non-micronized forms of the active substance at appropriate proportions, the selection of convenient excipients, their proportions; the proper distribution of the contents, the surface area, a suitable production process and a good formulation design.

In the present invention, the active ingredient is acemetacin or a pharmaceutically acceptable salt thereof. In the further sections, acemetacin term will be used as acemetacin or its pharmaceutically acceptable salt.

The present invention relates to a pharmaceutical formulation comprising active ingredients having different particle sizes. According to the claims, the acemetacin active substance is used in different particle sizes as micronized and non-micronized forms.

The "micronised" (performing "micronization") term indicates a very fine particle size in the micrometer range; these micronized particles are preferably produced by mechanical means and the crystal structure remains unchanged.

The present invention is a pharmaceutical formulation as bimodal in vitro release;
- immediate release unit comprising micronised acemetacin
- retard release unit comprising non-micronised acemetacin
with multiple units in a single dosage form providing sustained release.

The particle size of non-micronised acemetacin presented in retard release unit is D90 100 micrometers or less, D50 10 to 20 micrometers and D10 5 micrometers or less.

The particle size of micronised acemetacin presented in immediate release unit is d(90) 5 micrometers or less, D50 1 to 3 micrometers and D10 1 micrometers or less.

If D10, D50 and D90 are measured in volume unit; respectively represent 10%, 50% and 90% of particle size distribution.

According to the claims, the ratio of non-micronised acemetacin to micronised acemetacin is 2:1 to 5:1.

In another embodiment of the invention, the ratio of non-micronised acemetacin to micronised acemetacin is 2:1 to 3.5:1.

The invention preferably comprises acemetacin or equivalent salt in an amount ranging from 60 mg to 180 mg, preferably 60 mg to 90 mg, more preferably 90 mg.

In a preferred embodiment of the invention the filler is selected from the group consisting of lactose, microcrystalline cellulose, starch, pregelatinized starch, modified starch, calcium phosphate (dibasic and / or tribasic), calcium sulfate trihydrate or dihydrate, calcium carbonate, kaolin, cellulose, dextrose, dextrates, dextrin, sucrose, maltose, fructose, glucose, mannitol, maltol, sorbitol, xylitol, titanium dioxide (TiO₂). Preferably, the filler is lactose monohydrate and titanium dioxide.

In a preferred embodiment of the invention, the retard release unit containing non-micronized acemetacin and titanium dioxide; micronised acemetacin and lactose monohydrate, in the form of an immediate release unit.

The concentration of the release modifier (% weight/total weight), pH values of dissolution, the ratio of the proportions of the combined use of more than one release modifier are critical parameters, especially due to the variation in chemical and physical properties of the release modifiers.

In a preferred embodiment of the invention, the release modifier is at least one of the group consisting of starch, sugar, ethyl cellulose, cellulose acetate phthalate, hydroxypropylmethyl cellulose acetyl succinate, microcrystalline cellulose, acrylate copolymers (Eudragit derivatives), sodium carboxymethyl cellulose, polyvinyl acetate (Kollicoat® SR), hydroxypropylcellulose. Preferably, the release modifier is a mixture of at least two of the methacrylate copolymer derivatives.

The present invention relates to a pharmaceutical formulation having a retard release unit comprising combination of at least two methacrylic acid - acrylate copolymers and immediate release unit.

Release modifiers in the present invention include: the agent in the retard release unit permits release starting from pH 5.5 and pH 6.0. Thus, the formulation ensures that the active ingredient in the retard release unit does not dissolve prior to pH 5.0, except for the amount of active ingredient present in the immediate release unit. Eudragit L100-55 provides release from pH 5,5 and Eudragit L100 releases from pH 6.0. The ratio of Eudragit® L100 to Eudragit® L100-55 is from 40:60 to 60:40 by weight, more preferably 50:50 by weight.

The multiple unit dosage form is preferably filled into a soft or hard gelatin capsule or compressed into tablets. In a preferred embodiment of the invention, the dosage form is soft or hard gelatin capsules.

In a preferred embodiment of the invention, the multi unit pellet is a micro tablet, a granule, preferably more than one granule.

In a preferred embodiment of the invention, the granules are filled into a hard gelatin capsule.

The release profile of the oral multiple-unit dosage form is determined by the following procedure:
The dissolution test is carried out at pH 5.0 USP buffer, USP apparatus I (basket) at 37°C, 100 rpm rotation speed. Dissolution medium is; USP citric acid and sodium citrate buffer solution at pH 5.0.

Amounts of dissolved active ingredients over time were determined by HPLC.

In the present invention, the pharmaceutical formulation having an oral dosage form containing multiple units providing bimodal in vitro release in the above mentioned dissolution medium;
- at least 20% at first 15^{th} minutes
- between 30-40% at 1^{st} hour
presents a dissolution profile as such acemetacin release.

According to the present invention, the targetted dissolution profile is provided by means of different characteristic features of the multiple unit dosage form comprising immediate release and retard release units. More specifically, the targetted dissolution profile is provided by means of certain quantities of active substance quantities having different particle sizes and the amounts of excipients.

In the preferred embodiment of the invention, oral dosage forms containing multiple units are prepared. The plurality of units may contain the above-mentioned excipients in addition to the active substance. These excpients; one or more excipients selected from the group consisting of binders, lubricants, antistatic agents, dispersants, surface active agents, plasticizers (such as triacetin), glidants.

In a preferred embodiment of the invention, the binding is one of the group comprising gelatin, hydroxypropylcellulose (HPC), hydroxypropylmethyl cellulose (HPMC), methyl cellulose, polyvinyl pyrrolidone (PVP), sucrose, starch or the mixture thereof. Preferably, the binder is polyvinyl pyrrolidone.

In a preferred embodiment of the invention, said dosage forms comprise a pharmaceutically acceptable lubricant selected from at least one of the group comprising calcium stearate, glycerin, vegetable oil, magnesium stearate, mineral oil, polyethylene glycol, propylene glycol or the mixture thereof.

In a preferred embodiment of the invention said dosage forms comprise a pharmaceutically acceptable lubricant selected from the group consisting of kaolin, talc, silicon dioxide, and the mixture thereof.

In a preferred embodiment of the invention, at least one disintegrant selected from the group comprising alginate, croscarmellose sodium, crospovidone, sodium starch glycollate, pregelatinized starch is used in the production of such dosage forms.

In the preferred embodiment of the invention, at least one glidant selected from the group comprising colloidal silicon dioxide, magnesium stearate, starch, talc, is used in the production of such dosage forms.

In a preferred embodiment of the invention, said bimodal in vitro release-containing multiple-unit dosage form comprises:
- an immedite release unit comprising
   a. micronised acemetacin between 40% - 50% by weight
   b. lactose monohydrate between 30% - 40% by weight
   c. crospovidon between 1% - 5% by weight
   d. povidone between 5% - 10% by weight
   e. aerosil between 0.1% - 1% by weight
   f. talc between 0.1% - 5% by weight
   g. magnesum stearate between 0.1% - 5% by weight
- a retard release unit comprising (w/w% of corresponding unit)
   a. non-micronised acemetacin between 30% - 40% by weight
   b. titanium dioxide between 40% - 50% by weight
   c. crospovidon between 10% - 5% by weight
   d. povidone between 5% - 10% by weight
   e. acrylate copolymers between 10% - 20% by weight
   f. triacetin between 0.1% - 5% by weight
   g. aerosil between 0.1% - 1% by weight
   h. talc between 0.1% - 5% by weight
   i. magnesium stearate between 0.1% - 5% by weight

Another embodiment of the invention is a method for preparing capsule dosage forms with multiple units by the following steps. In the preferred embodiment of the invention, the retard release formulation is prepared by double granulation method.
a) to prepare retard release unit
   i. prepare the pre-mix by mixing non-micronised acemetacin, crospovidon, titanium dioxide,
   ii. dissolve povidon in ethyl alcohol,
   iii. granulation of step i with the solution prepared in step ii,
   iv. drying the granule to obtain a granule with a maximum mositure value of 2%,
   v. sifting the dried granules,
   vi. dissolving acrylate copolymers in ethyl alcohol and folowing mixing by adding triacetin,
   vii. e-granulate the dried granules obtained in step v by mixing with the solution in step vi,
   viii. drying the granule in step vii to obtain a granule with a maximum mositure value of 2%,
   ix. sifting the dried granules obtained in step vii,
   x. adding aerosil, talc and magnesium stearate to the dried granules in step ix seperately and mix to obtain homojenous mixture.
b) to prepare immediate release unit
   i. to prepare powder mixture by mixing micronised acemetacin, crospovidon, lactose monohydrate,
   ii. dissolving povidone in ethyl alcohol,
   iii. granulation of step i with the solution prepared in step ii,
   iv. drying the granule to obtain a granule with a maximum mositure value of 2%,
   v. sifting the dried granules,
   vi. adding aerosil, talc and magnesium stearate to the dried granules in step ix seperately and mix to obtain homojenous mixture.

The final process to obtain finished product is the step of filling the capsules with the granules obtained in unit a and unit b.

In a preferred embodiment of the invention, the retard release unit is prepared by double granulation method. The present invention has multiple unit dosage forms comprising a retard release unit to which the double granulation process is applied and an immediate release unit to which a single granulation process is applied. The following examples are presented for better illustration of the invention and are not to be limited with the examples presented below.

### Examples

### Example 1 - Composition and preparation of multiple unit dosage forms

Said pharmaceutical formulation comprises immediate release and retard release units comprising acemetacin and pharmaceutically acceptable excipients. The table below (Table 1) contains the formulation information of oral dosage forms containing the developed multiple-units. Multiple-unit forms are in the form of granules.

**Table 1. Oral Pharmaceutical Formulation with Multiple Units**

| | **weight %/total weight** |
|---|---|
| **Immediate release unit** | |
| Acemetacin (micronised) | 45-50 |
| Lactose monohydrate | 35-40 |
| Crospovidon | 3 -5 |
| Povidone | 5-10 |
| Aeorosil | 0,1-1 |
| Talc 1-4 | |
| Magnesium stearate | 1- 4 |
| Ethyl alcohol | q.s. |
| Total weight of immediate release unit is between 40 mg to 70 mg. | |

| **Retard release unit** | |
|---|---|
| Acemetacin (non-micronised) | 35 - 40 |
| Titanium dioxide | 28 - 35 |
| Crospovidon | 2-5 |
| Povidone | 5 - 7 |
| Acrylate copolymers | 10-20 |
| Triacetine | 2-5 |
| Aerosil | 0,1 - 0,6 |
| Talc | 1-3 |
| Magnesium stearate | 1-3 |
| Ethyl alcohol | k.m. |
| Total weight of retard release unit is between 150 mg to 185 mg. | |

| | |
|---|---|
| ^{∗} The ratio between acrylate copolymers above is 50:50 by weight. | |

The ratios of micronized and non-micronized acemetacin of the multiple unit oral pharmaceutical dosage form examples are given in Table 1 as Example I, Example II, Example III.

**Table 2. The ratios of active substance in examples by weight**

| | **Example I** | **Example II** | **Example III** |
|---|---|---|---|
| Acemetacin (non-micronised) : Acemetacin (micronised) | 2:1 | 2.6:1 | 3.5:1 |

The retard release unit for preparing capsule dosage forms with multiple units is prepared in more detail as follows:
The micronized acemetacin, crospovidone, titanium dioxide is mixed for 10 minutes to obtain a mixture. Povidone is dissolved in ethyl alcohol. The granule is obtained by adding soluble povidone to the obtained dry mixture. The granule is dried at 40 - 60°C so that it has a maximum moisture content of 2%, it is sifted by passing it through 800 µm sieve. The methacrylate copolymer combination is dissolved in ethyl alcohol and then mixed by adding triacetin. The resulting mixture is mixed with the dry granule in the mixer. The granule is dried at 40 - 60°C so that it has a maximum moisture content of 2%, it is sifted by passing it through 800 µm sieve. The dry granules are mixed for 3 to 5 minutes with the addition of aerosil, talc and magnesium stearate, respectively.

For the preparation of capsule dosage forms containing multiple units, the immediate release unit is prepared in detail as follows:
Micronised acemetacin, crospovidone, lactose monohydrate are mixed for 10 minutes. Povidone is dissolved in ethyl alcohol. The granule is obtained by adding povidone which is soluble in ethyl alcohol on the powder mixture. The granule is dried at 40 - 60°C so that it has a maximum moisture content of 1.5%, it is sifted by passing it through 800 µm sieve. The dry granules are mixed for 3 to 5 minutes with the addition of aerosil, talc and magnesium stearate, respectively.

The granules obtained in the preparation of the above-mentioned retard release and immediate release units are filled into a hard gelatin capsule.

Examples (IV, V, VI) which contain multiple units developed and which represents the subject invention (I, II, III), multiple units and which are outside the scope of the invention (IV, V, VI) (Examples VI, VII, VIII); the effective parameters in the dissolution profile were compared in the table (Table 3) below. All samples are in granular form and filled in a capsule.

**Table 3. Examples**

| | **Ex. I** | **Ex. II** | **Ex. III** | **Ex. IV** | **Ex. V** | **Ex. VI** | **Ex. VII** | **Ex. VIII** | **Ex. IX** |
|---|---|---|---|---|---|---|---|---|---|
| **Ratio of non-micronised acemetacin to micronised acemetacin** | 2 | 2.6 | 3.5 | 5 | 8 | X | X | 1.5 | 1.5 |
| **Double granulation process in retard release unit** | + | + | + | + | + | + | + | + | - |

Formulations containing single units of Examples VI, VII and VIII of Table 3 are prepared by a similar method to the above described delayed release unit preparation method.

### Example 2 - Dissoluion Test

Dissolution tests were performed on the formulations in Table 3 in the direction of the following parameters. Each capsule contains 90 mg of acemetacin active ingredient.

**Table 4. Parameters of Disolution Test**

| | |
|---|---|
| Medium | pH 5.0 USP buffer |
| Volume | 900 mL |
| Apparatus | Basket |
| Temperature | 37°C |
| Rotaion | 100 rpm |
| Analysis method | HPLC |

### Table 3 - Comparative Results of Dissolution Test

The dissolution test results stated in the Table 4 below were obtained by performing dissolution test in pH 5.0 USP buffer medium.

**Table 4. Dissolution Test, pH 5.0 USP Buffer (Example I-IX)**

| **Time (min.)** | **% Drug Release** | | | | | | | | |
|---|---|---|---|---|---|---|---|---|---|
| | ***Example I*** | ***Example II*** | ***Example III*** | ***Example IV*** | ***Example V*** | ***Example VI*** | ***Example VII*** | ***Example VIII*** | ***Example IX*** |
| **0** | 0.0 | 0.0 | 0.0 | 0.0 | 0.0 | 0.0 | 0.0 | 0.0 | 0.0 |
| **5** | 16.4 | 16.0 | 11.1 | 8.4 | 7.9 | 2.7 | 1.1 | 2.4 | 6.0 |
| **10** | 25.6 | 20.4 | 19.1 | 15.6 | 12.7 | 6.6 | 3.8 | 4.6 | 15.1 |
| **15** | **29.1** | **23.4** | **22.6** | **18.6** | **15.0** | **8.8** | **5.3** | **6.0** | **19.2** |
| **20** | 31.5 | 25.6 | 25.0 | 20.4 | 16.8 | 10.5 | 6.5 | 7.1 | 23.2 |
| **30** | 34.6 | 28.2 | 26.8 | 22.9 | 19.2 | 13.1 | 8.3 | 8.9 | 26.2 |
| **45** | 37.2 | 30.9 | 29.1 | 25.4 | 21.6 | 16.0 | 10.4 | 11.1 | 28.1 |
| **60** | **38.9** | **32.8** | **30.6** | **27.3** | **23.4** | **18.2** | **12.3** | **12.9** | **29.0** |

## Claims

1. An oral pharmaceutical formulation with bimodal in vitro release comprising acemetacin or a pharmaceutically acceptable salt thereof wherein the composition is composed of
a. an immediate-release unit comprising micronised acemetacin or its pharmaceutically acceptable salt thereof, wherein the particle size of micronised acemetacin is d(90) 5 µm or less, d(50) 1 to 3 µm, i, d(10) less than 1 µm,
b. a retard-release unit comprising non-micronised acemetacin or its pharmaceutically acceptable salt thereof and at least two release modifiers, wherein particle size of non-micronised acemetacin is d(90) 100 µm or less, d(50) 10 to 20 µm , d(10)less than 5 µm, wherein the multiple-unit dosage form presents a dissolution profile at least 20% after 15 minutes and 30%-40% after one hour in conditions of Type I apparatus (basket), 100 rpm at 37°C, 900 ml USP buffer pH 5.0 with the w/w ratio of non-micronised acemetacin to micronised acemetacin of 2:1 to 5: 1.

2. An oral pharmaceutical composition according to claim 1 wherein release modifier is selected from the group comprising starch, sugar, ethyl cellulose, acetate phthalate, hydlroxypropylmethyl cellulose acetyl succinate, microcrystalline cellulose, methacrylate copolymers , sodium carboxymethyl cellulose, polyvinyl acetate, hydroxypropylcellulose and the mixture thereof

3. An oral pharmaceutical composition according to claim 2, wherein the release modifier is Poly(methacylic acid-co-methyl acrylate) .

4. An oral pharmaceutical composition according to claim 2, wherein the release modifier is Poly(methacylic acid-co-ethyl acrylate).

5. An oral pharmaceutical composition according to claim 2, wherein the ratio of Poly(methacylic acid-co-methyl acrylate) to Poly(methacylic acid-co-ethyl acrylate) is from 40:60 to 60:40 by weight, more preferably 50:50 by weight

6. An oral pharmaceutical composition according to any of the preceding claims wherein release modifier mixture comprising a release modifier dissolves at pH 5.5 and a release modifier dissolves at pH 6.0 is in the total amount of 10% - 20% by weight.

7. An oral pharmaceutical composition according to any of the preceding claims wherein the composition also comrises one or more excipients.

8. An oral pharmaceutical composition according to claim 7, the composition comprises multiple units.

9. An oral pharmaceutical composition according to claim 8, the pharmaceutical compositon is in the dosage form of soft or hard gelatine capsule comprising tablet, film coated tablet, micro tablet, pellet, powder, granule or combinations thereof.

10. An oral pharmaceutical composition according to claim 9, the composition is in the dosage form of hard gelatine capsule comprising more than one granules.

11. An oral pharmaceutical composition according to any of the preceding claims wherein the composition comprises 60 mg to 180 mg acemetacin or equivalent amount of therapeutically acceptable salt or the mixture thereof.

12. An oral pharmaceutical composition according to any of the preceding claims wherein it is indicated in the treatment of osteoarthritis, rheumatoid arthritis and ankylosing spondylitis, and acute gouty arthritis, acute musculoskeletal pain, postoperative pain, and dysmenorrhea.

## Patentansprüche

1. Orale pharmazeutische In-vitro-Formulierung mit bimodaler Freisetzung, umfassend Acemetacin oder ein pharmazeutisch akzeptables Salz davon, wobei die Zusammensetzung zusammengesetzt ist aus:
a. eine Einheit zur sofortigen Freisetzung, umfassend mikronisiertes Acemetacin oder ein pharmazeutisch akzeptables Salz davon, wobei die Teilchengröße des mikronisierten Acemetacin d(90) 5 µm oder weniger, d(50) 1 bis 3 µm, d(10) weniger als 1 µm beträgt,
b. eine Einheit mit verzögerter Freisetzung, umfassend nicht-mikronisiertes Acemetacin oder sein pharmazeutisch akzeptables Salz und mindestens zwei Freisetzungsmodifikatoren, wobei die Teilchengröße des nicht-mikronisierten Acemetacin d(90) 100 µm oder weniger, d(50) 10 bis 20 µm, d(10) weniger als 5 µm beträgt wobei die Mehrfacheinheits-Dosierungsform ein Auflösungsprofil von mindestens 20 % nach 15 Minuten und 30 % bis 40 % nach einer Stunde unter Typ 1 (Korb)-Apparat-Bedingungen, 100 U/min bei 37°C, 900 ml USP-Puffer pH 5,0 mit dem w/w-Verhältnis von nicht-mikronisiertem Acemetacin zu mikronisiertem Acemetacin von 2: 1 bis 5:1.

2. Orale pharmazeutische Zusammensetzung nach Anspruch 1, wobei der Freisetzungsmodifikator ausgewählt ist aus der Gruppe bestehend aus Stärke, Zucker, Ethylcellulose, Acetatphthalat, Hydroxypropylmethylcelluloseacetylsuccinat, mikrokristalliner Cellulose, Methacrylatcopolymeren, Natriumcarboxymethylcellulose, Polyvinylacetat, Hydroxypropylcellulose und Kombinationen davon.

3. Orale pharmazeutische Zusammensetzung nach Anspruch 2, wobei der Freisetzungsmodifikator ist ein Poly(methacrylsäure-co-methylacrylat).

4. Orale pharmazeutische Zusammensetzung nach Anspruch 2, wobei der Freisetzungsmodifikator ist Poly(methacrylsäure-ethylcoacrylat).

5. Orale pharmazeutische Zusammensetzung nach Anspruch 2, wobei das Gewichtsverhältnis von Poly(methacrylsäure-co-methylacrylat) zu Poly(methacrylsäure-co-ethylacrylat) 40:60 bis 60:40, bevorzugter 50:50, beträgt.

6. Orale pharmazeutische Zusammensetzung nach einem der vorhergehenden Ansprüche, wobei das Gemisch von Freisetzungsmodifikatoren, umfassend einen Freisetzungsmodifikator, der sich bei pH 5,5 auflöst, und einen Freisetzungsmodifikator, der sich bei pH 6,0 auflöst, in einer Gesamtmenge von 10 bis 20 Gew.-% vorliegt.

7. Orale pharmazeutische Zusammensetzung nach einem der vorhergehenden Ansprüche, wobei die Zusammensetzung auch einen oder mehrere Hilfsstoffe umfasst.

8. Orale pharmazeutische Zusammensetzung nach Anspruch 7, wobei die Zusammensetzung mehrere Einheiten umfasst.

9. Orale pharmazeutische Zusammensetzung nach Anspruch 8, wobei die pharmazeutische Zusammensetzung in der Darreichungsform einer weichen Hartgelatinekapsel vorliegt, die eine Tablette, einen beschichteten Film, eine Mikrotablette, ein Pulver, ein Granulat oder Kombinationen davon umfasst.

10. Orale pharmazeutische Zusammensetzung nach Anspruch 9, wobei die Zusammensetzung in der Darreichungsform einer Hartgelatinekapsel vorliegt, die mehr als ein Granulat umfasst.

11. Orale pharmazeutische Zusammensetzung nach einem der vorhergehenden Ansprüche, wobei die Zusammensetzung 60 mg bis 180 mg Acemetacin oder eine äquivalente Menge eines therapeutisch akzeptablen Salzes oder eine Kombination davon umfasst.

12. Orale pharmazeutische Zusammensetzung nach einem der vorhergehenden Ansprüche, wobei sie bei der Behandlung von Osteoarthritis, rheumatoider Arthritis und ankylosierender Spondylitis und akuter Gichtarthritis, akuten muskuloskelettalen Schmerzen, postoperativen Schmerzen und Dysmenorrhoe angezeigt ist.

## Revendications

1. Une formulation pharmaceutique orale à libération bimodale in vitro comprenant de l'acémétacine ou un sel pharmaceutiquement acceptable de celle-ci, dans laquelle la composition est composée de :
a. une unité à libération immédiate comprenant de l'acémétacine micronisée ou son sel pharmaceutiquement acceptable, dans laquelle la taille des particules d'acémétacine micronisée est d(90) 5 µm ou moins, d(50) 1 à 3 µm, d(10) moins de 1 µm,
b. une unité à libération prolongée comprenant de l'acémétacine non micronisée ou son sel pharmaceutiquement acceptable et au moins deux modificateurs de libération, dans laquelle la taille des particules d'acémétacine non micronisée est d(90) 100 µm ou moins, d(50) 10 à 20 µm, d(10) moins de 5 µm, dans laquelle la forme posologique à unités multiples présente un profil de dissolution d'au moins 20 % après 15 minutes et de 30 % à 40 % après une heure dans des conditions d'appareil de type 1 (panier), 100 rpm à 37°C, 900 ml de tampon USP pH 5,0 avec le rapport p/p d'acémétacine non micromisée à l'acémétacine micromisée de 2:1 à 5:1.

2. Une composition pharmaceutique orale selon la revendication 1, dans laquelle le modificateur de libération est choisi dans le groupe comprenant l'amidon, le sucre, l'éthylcellulose, l'acétate phtalate, l'acétyle succinate d'hydroxypropylméthylcellulose, la cellulose microcristalline, les copolymères de méthacrylate, la carboxyméthylcellulose sodique, l'acétate de polyvinyle, l'hydroxypropylcellulose et leur combinaison.

3. Une composition pharmaceutique orale selon la revendication 2, dans laquelle le modificateur de libération est un poly(acide méthacrylique-co-acrylate de méthyle)

4. Une composition pharmaceutique orale selon la revendication 2, dans laquelle le modificateur de libération est le poly(acide méthacrylique-co-acrylate d'éthyle).

5. Une composition pharmaceutique orale selon la revendication 2, dans laquelle le rapport du poly(acide méthacrylique-co-acrylate de méthyle) au poly(acide méthacrylique-co-acrylate d'éthyle) est de 40:60 à 60:40 en poids, plus préférablement de 50:50 en poids.

6. Une composition pharmaceutique orale selon l'une quelconque des revendications précédentes, dans laquelle le mélange de modificateurs de libération comprenant un modificateur de libération se dissolvant à pH 5,5 et un modificateur de libération se dissolvant à pH 6,0 est dans la quantité totale de 10% - 20% en poids.

7. Une composition pharmaceutique orale selon l'une quelconque des précédentes revendications, dans laquelle la composition comprend également un ou plusieurs excipients.

8. Une composition pharmaceutique orale selon la revendication 7, la composition comprend de multiples unités.

9. Une composition pharmaceutique orale selon la revendication 8, la composition pharmaceutique est sous la forme de dosage d'une capsule de gélatine dure molle comprenant un comprimé, un film enrobé, un micro comprimé, une poudre, un granulé ou des combinaisons de ceux-ci.

10. Une composition pharmaceutique orale selon la revendication 9, la composition est sous la forme de dosage d'une capsule de gélatine dure comprenant plus d'un granule.

11. Une composition pharmaceutique orale selon l'une quelconque des précédentes revendications dans laquelle la composition comprend 60 mg à 180 mg d'acémétacine ou une quantité équivalente de sel thérapeutiquement acceptable ou de leur combinaison.

12. Une composition pharmaceutique orale selon l'une quelconque des précédentes revendications, dans laquelle elle est indiquée dans le traitement de l'arthrose, de la polyarthrite rhumatoïde et de la spondylarthrite ankylosante, et de l'arthrite goutteuse aiguë, de la douleur musculosquelettique aiguë, de la douleur postopératoire et de la dysménorrhée.
